# EUROPEAN PATENT SPECIFICATION

(11) **EP 2 341 971 B1**
(45) Date of publication and mention of the grant of the patent: **20.03.2019**
(21) Application number: 09823895.9
(22) Date of filing: 03.07.2009
(51) Int. Cl.: A61M 16/04, A61B 17/28

(54) **DEVICE FOR TRACHEOTOMY**
TRACHEOTOMIE-VORRICHTUNG
DISPOSITIF DE TRACHÉOTOMIE

(30) Priority: 31.10.2008 SE 0802321
(43) Date of publication of application: 13.07.2011
(73) Proprietor: SafeTrach AB, 131 41 Nacka (SE)
(72) Inventor: MARGOLIN, Gregory, 116 41 Stockholm (SE); KARLING, Jonas, 131 41 Nacka (SE)
(74) Representative: Ström & Gulliksson AB
(86) International application number: PCT/SE2009/050873
(87) International publication number: WO 2010/050875

(56) References cited:
- WO-A1-2007/018472
- WO-A1-2007/018472
- GB-A- 2 452 400
- US-A- 4 832 020
- US-A- 5 203 320
- US-A1- 2007 017 527

## Description

### Field of the invention

The present invention pertains to the field of tracheotomy, i.e. creation of an opening in the windpipe (trachea) just below the larynx towards the surrounding air.

### Background of the invention

Tracheotomy is one of the most common life saving operations that is done within medical care. A tracheotomy involves opening a hole in the neck part of the trachea. The term tracheostomy is sometimes used interchangeably with tracheotomy. Strictly speaking, however, tracheostomy usually refers to the opening itself while a tracheotomy is the actual operation.

Some common indications for a tracheotomy are:
- Respiratory obstruction - an obstruction of the upper respiratory passages;
- Respiratory failure / respiratory insufficiency;
- Respiratory paralysis;
- Removal of retained secretion;
- Reduction of dead space.

About 15 % of all patients treated in intensive care units need a tracheotomy.

Tracheotomy was already performed in ancient times. Of course procedure was at that time associated with high risk and was consequently rarely performed up to the beginning of the twentieth century. At that time Chevalier Jackson described routines for performing the procedure, which lead to making the procedure safer and even more common. He described what is called *open tracheotomy.*

Open tracheotomy is a surgical operation that is performed in an operating theatre by a surgical team that consists of an ear, nose and throat surgeon, and preferably an assisting surgeon, as well as a theatre nurse, an anesthesiologist, and an anesthesia nurse and also a nurse's assistant. A sterile sheet environment is necessary. It is preferred to perform the operation on an intubated patient in anesthesia together with supplementary local anesthesia in the surgical area. During acute situations, when intubation is difficult or impossible to be done, the surgical operation is performed with the patient awake, in local anesthesia. A horizontal incision is made in the skin above the breastbone (above the suprastenal notch). Then, a cut is performed sharply through the platysma. The straight neck muscles are pulled aside. However, the thyroid gland isthmus should preferably be avoided, but if it is in the way, it is split and bound up. The trachea is then opened with a horizontal incision for adults and with a vertical incision for children. A tracheal tube is inserted and connected to a respirator. Then the wound is closed with sparse sutures and finally the tube is fixed with sutures and tracheal bands.

During the last thirty years even so called *percutaneous tracheotomy* has been established. This technique became widely spread despite highly frequent complications. The frequency of complications could be reduced considerably by introducing an endoscopical guidance. The result of this development was that the indications for a tracheotomy changed and specialists who perform a tracheotomy, who earlier exclusively were ear, nose and throat specialist, include now even intensive care physicians.

Furthermore, percutaneous tracheotomy was already described during the sixties, but was not accepted as a secure surgical operation before the eighties when the group of Ciaglia introduced dilatators. The surgical operation may be performed bedside on an intensive care unit in anesthesia by two trained physicians, whereof one carries out the surgical operation and the other inspects the trachea from the inside by means of a bronchoscope. Moreover, an anesthesia nurse is necessary.

The surgical area is locally anesthetized and a horizontal incision is made in the skin. A cuffed endotracheal tube is pulled up against the vocal cords. The tracheal wall is, under inspection via a bronchoscope, punctured with a needle through the endotracheal tube. Subsequently a guidewire is introduced. On the latter a dilatator having increasing gauge is then introduced. Finally, a tracheal tube, which is slipped on a dilatator, is introduced into the tracheostoma that is produced. The dilatator is withdrawn and the tracheal tube is positioned. The method does not leave any wounds that have to be sutured, and the tracheal tube is fixed in the same manner as with open technique. The patient's head must be kept centered, such that the puncture does not end up aside of the trachea. The position of the percutaneous tracheotomy is also located 2cm above the breastbone.

The surgical operation necessitates staff that is acquainted with this technique. A number of surgical operations under experienced supervision are necessary before it can be performed independently. The frequency of complications is rather high and comprises amongst others pneumothorax, perforation of the rear tracheal wall and oesophagus, as well as bleedings. The procedure is complicated with patients who have a short and thick neck, and patients having enlarged thyroid gland. It is not recommended for children.

The percutaneous technique has eliminated drawbacks that an open tracheotomy involves, including the need of an operation theatre and staff intensive effort, and that it leaves an open wound that can become an infection site. Furthermore, it also puts heavy demands on surgical skill training. Moreover, the open technique is burdened with the risk of surgical complications.

Hence, there is a need for an improved tracheotomy technique. In particular a device allowing for increased flexibility, cost-effectiveness, as well as patient safety and comfort would be advantageous.

### Summary of the invention

Accordingly, the present invention preferably seeks to mitigate, alleviate or eliminate, amongst others, one or more of the above-identified, or other, deficiencies and disadvantages in the art, singly or in any combination, and solves for instance the above mentioned problems at least partly by providing a device according to the appended patent claims.

According to one aspect of the invention, a device is provided for facilitating tracheotomy, comprising a first branch, adapted to be introduced into the trachea of a patient, and a second branch, adapted to be arranged at the outside of the neck with the distal end thereof located adjacent to a site that is intended for a tracheostoma, wherein the end of the second branches comprises a guide for a sharp object, and the end of the first branch comprises a receiving means, and wherein the first branch and the second branch are joined to each other in such a manner that the guide is directed towards the receiving means, whereby, when the sharp object penetrates the skin and tracheal wall in order to carry out the tracheostoma, the sharp object is directed towards the receiving means, characterized in that said first branch comprises a directing means, adapted to slidingly engage with a tube in trachea, said directing means being located close to the distal end of said first branch, for directing said first branch along said tube.

The present disclosure also describes a method of carrying out a tracheotomy, comprising introducing a first branch of a device for facilitating tracheotomy down a trachea of a patient, such that the distal end thereof is located below the larynx, and arranging a second branch of the device on the outside of the neck with the distal end thereof located adjacent to the site that is intended for a tracheostoma, wherein the distal end of one of the first and second branches comprises a guide for a sharp object, and the distal end of the other branch comprises a receiving means, and wherein the first and second branch are joined to each other in such a manner that the guide is directed towards the receiving means, as well as penetrating the skin and tracheal wall with said sharp object in order to carry out the tracheotomy by directing the sharp object towards the receiving means though said guiding device.

### Brief description of the drawings

These and other aspects, features and advantages of which the invention is capable of will be apparent and elucidated from the following description of embodiments of the present invention, reference being made to the accompanying drawings, in which
Figure 1 is a schematic illustration of a device that is configured to be used during creation of a tracheostomy in form of a forceps according to an embodiment;
Figure 2 is a schematic illustration of a device for facilitating tracheotomy in form of two connectable branches according to an embodiment;
Fig. 3 is a perspective view of an embodiment of the present invention;
Fig. 4 is a perspective view of the embodiment according to Fig. 3, in cooperation with an endotracheal tube;
Fig. 5 is a side view of the embodiment according to Fig. 3;
Figure 6A and 6B are schematic illustrations of a further device for facilitating tracheotomy in two different states;
Figure 7A and 7B are schematic illustrations illustrating the release of a locking mechanism of the device shown in Figures 6A and 6B; and
Figure 8 is a schematic illustration illustrating the two states shown in Figure 6A and 6B, respectively, with an alternative locking mechanism.

### Detailed description of embodiments of the invention

Embodiments of the invention are described in more detail below, being illustrated in the attached drawings.

More precisely, a device has been developed that make use of all the advantages that percutaneous tracheotomy offers, but which dramatically simplifies and standardizes the technique and eliminates many known risk factors, which the percutaneous technique involves.

The previously known techniques, which are described in the background section, are more difficult to carry out than may be concluded from the above and from what is described in the literature. This applies also for instance to the demonstration CD-Rom and user manual of Cook Critical Care Inc., Bloomington, Indiana, which follows with their percutaneous kit.

The different steps, which are performed during the percutaneous tracheotomy, each imply the following issues to be considered:
1. Where should the tracheotomy be made? In the general instructions it is written that it should be made 2cm above the upper edge of the breastbone. On thick and short necks and with swellings after a long intubation time, it is difficult to find that spot. In Cooks instructions it is recommended that a bronchoscope is introduced through the endotracheal tube and that the incision is made where the light source of the bronchoscope is. However, on a thicker neck it is not possible to identify the light source before an incision is made through the skin. As long as the bronchoscope is inside the endotracheal tube, it is not possible to see a concentrated light source. In order to be able to use the light source in practice as a point of identification, the endotracheal tube firstly has to be pulled up, in such a manner that the tip of the bronchoscope appears outside of the tubes' bottom edge. This necessitates a certain degree of caution in order not to extubate the patient.
   In accordance with an embodiment of the present invention, a device similar to a pair of pliers/forceps is used, having a first branch that is designed to be introduced down into the trachea, and a second branch that is located on the outside of the neck, opposite to the first branch. The first branch terminates in a protecting plate, and the other branch is provided with a guiding tube for a tracheal puncturing device, such as an obturator with a sharp tip or a needle. The first branch is introduced down into the trachea along an endotracheal tube. The first branch is guided along the outside of the endotracheal tube, by the aid of a directing means, close to the distal end of the first branch. The directing means is adapted to grip around the endotracheal tube, such that the first branch is slidably displaceable along the endotracheal tube. The directing means may be two hooks with the concave surfaces thereof facing each other. The two hooks may the enclose the endotracheal tube already positioned in the trachea of the patient, whereafter the distal end of the first branch may be slidably displaced along the endotracheal tube, into the position of tracheotomy. In this way, the first branch may be controllably inserted into the trachea, without jeopardizing to injure the trachea of the patient. The first branch rests in this case against the front side of the endotracheal tube and will be centered due to the fact that the larynx is triangular with its one apex directed forward.
   In one embodiment the directing means comprises only one hook. In this embodiment the extension of said hook is preferably somewhat longer than the individual hooks, when the directing means comprises two hooks, to ensure satisfactory engagement with the tube.
   In a further embodiment, the directing means is a ring, adapted to encircle the endotracheal tube. The endotracheal tube is then inserted into said ring, where after the first branch may be directed by the tube into trachea.
   Embodiments of the device for facilitating tracheotomy are described in more detail further below in this specification.
   By means of the forceps like technique, enabled by the embodiment, an inner branch of the device is introduced along the endotracheal tube, while the end of the outer branch during the process indicates the position of the end of the inner branch.
   With the conventional tracheotomies, the determination of level becomes highly sensitive, but thanks to the forceps technique according to the embodiment, accuracy becomes less important because one is always positive about being well centered inside the trachea, see the explanation given below, and one always knows that the protection plate of the inner branch will be targeted during the penetration.
   If, after all, higher precision is desired concerning how long below the vocal cords the tracheostoma is to be positioned, - which actually is the most important measure in this context, but which previously has not been indicated because the conventional techniques did not offer this possibility -the forceps technique enabled by the embodiment offers the following opportunities:
   - The inner branch of certain embodiments of the tracheotomy facilitating device may have a scale, e.g. centimeter scale, similar to that found on endotracheal tubes. Thus a measure is provided that facilitates the whole tracheotomy process, as for instance a bronchoscopic surveillance of the process is omittable.
   - The endotracheal tube is kept with the cuff below the position of tracheotomy, which guarantees safe ventilation during the tracheotomy procedure, since there is no need to insert the first branch into the endotracheal tube.
   - The distance from the vocal cords is determinable by means of the scale of the inner branch and knowledge about the length of the endotracheal tube, as indicated above.
2. Another difficulty that previously known percutaneous techniques present, is to make the puncture strictly in the centerline of the tracheal wall. Namely, since it may be difficult to find the centerline of patients having a thick neck. When the centerline has been found, after all, it is important to introduce the puncture needle strictly saggital through the wall, both laterally as well as vertically. If this fails, the needle may slide down the side of the trachea and injure vessels, nerves or puncture the lung (pneumothorax) or injure the oesophagus.
   This problem is eliminated by means of the forceps technique described herein, as the lower part of the inner branch of the tracheotomy facilitating device always will be arranged in the middle of the trachea as a consequence of it being arranged along the endotracheal tube, which itself is arranged centered in the trachea by means of a cuff. The direction of the tube and, axially of the inner branch, is determined by means of a facemask or the like, which is used for first aid.
   The design of the forceps guarantees that the puncture is made correctly with regard to height, due to the angle of the guide at the tip of the outer branch.
3. In case of a puncture created by means of the previously known percutaneous technique, the penetration of the tracheal wall by a needle has to be thoroughly monitored via a bronchoscope, in order to know if one has penetrated the front wall and that the needle shall not travel too deeply and injure the rear wall. A requirement is that both the endotracheal tube and the bronchoscope are pulled up above the puncture site. This requires a certain amount of experience of the person handling the bronchoscope.

Such supervision is not necessary thanks to the tracheotomy technique enabled by the present embodiment, because a sharp object, like an obturator or a needle, and optionally a dilatator, is guided through the tip of the outer branch and is always directed towards a receiving device of the inner branch at a well defined location within the trachea, e.g. facing towards a bowl formed plate of the inner branch after penetration. The sharp object may engage the receiving device or it may be arranged so that it stops at the end of penetration a certain distance from the receiving device. For instance, the tip of the sharp object may be stopped by a suitable seat in the guiding means of the outer branch. In the case of tracheotomy it is important that the outer wall of the trachea is penetrated in order to create the tracheostoma. However, in certain cases it may be desired to provide a limitation of the stroke of the puncturing device towards the receiving device.

When the dilatator has penetrated the tracheal wall and is put in place, the outer branch may be disconnected. The dilatation of the wall may then be done by means of the dilatator including an introduction of a tracheal port, also called trachport.

A further alternative procedure is to arrange the needle in such a way that it penetrates the trachea outwardly from the inside. It is thus also possible to perform the dilatation from inside outwardly with a dilatator.

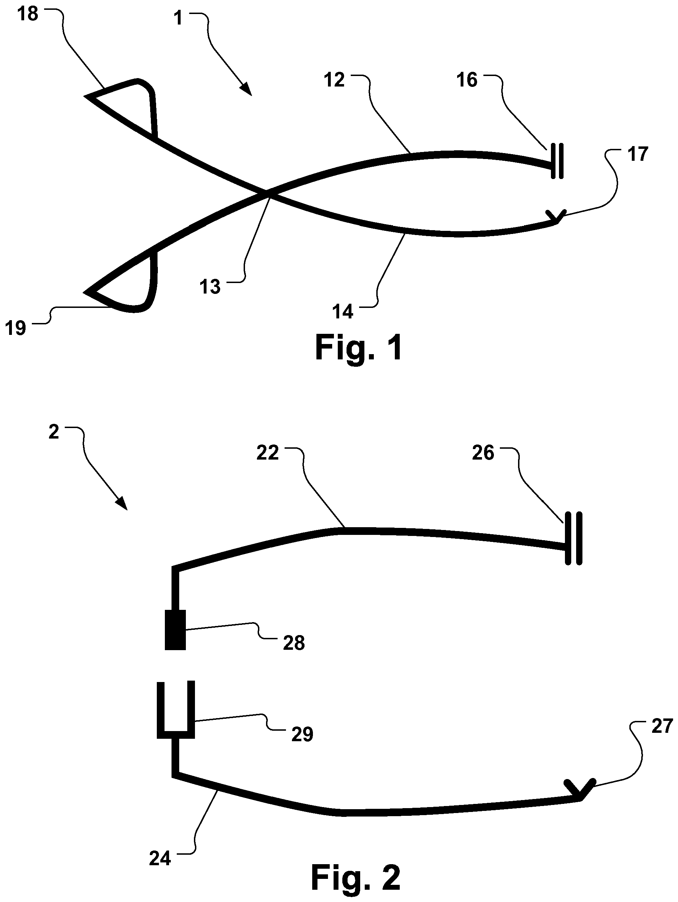

Now turning to the embodiments shown in the Figures, an embodiment of a tracheotomy facilitating device according to Figure 1, comprises a device 1 similar to a pair of forceps 1, having a first branch 14 that is designed to be introduced down into the trachea, and a second branch 12 that is located on the outside of the neck, opposite to the first branch 14. The two branches are pivoted around a common axis 13. The first branch 14 terminates distally in a protecting plate 17, and the other branch is distally provided with a guiding tube 16 for a tracheal puncturing device, such as an obturator or a needle. In use of device 1, the first branch is introduced down into the trachea along an endotracheal tube. This procedure is made easy by two handles 18, 19 on the proximal ends of forceps 1, respectively.

A further embodiment of the device is illustrated in Figure 2. This alternative device comprises two separate branches that cannot move pivotally in relation to each other, but which are pushed into one another at each proximal end 28, 29, respectively, until the opposite ends meet, i.e. the two branches may not be turned laterally. More precisely, this tracheotomy facilitating device 2 comprises a first branch 24 that is designed to be introduced down into the trachea, and a second branch 22 that is located on the outside of the neck, opposite to the first branch 24. The first branch 24 terminates in a protecting plate 27, and the other branch is provided with a guiding tube 26 for a tracheal puncturing device, such as an obturator or a needle. The two branches 22, 24 are connectable to each other at ends 28, 29, as indicated by the double-headed arrow in Figure 2.

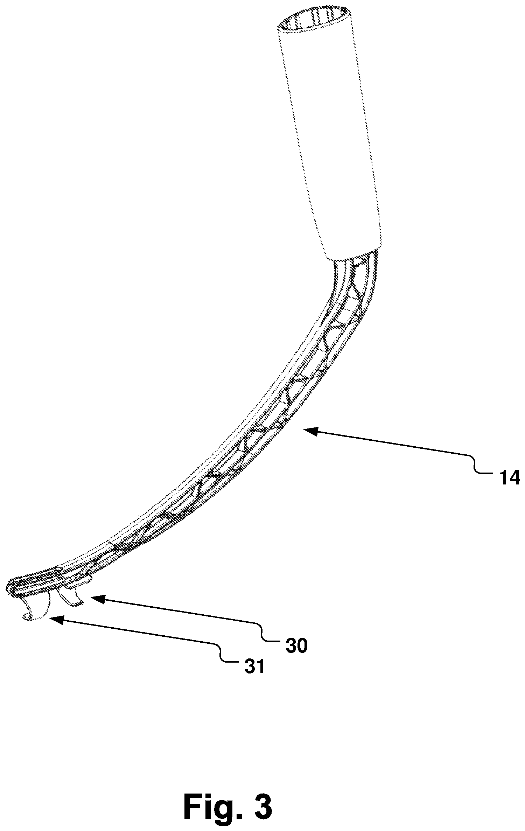

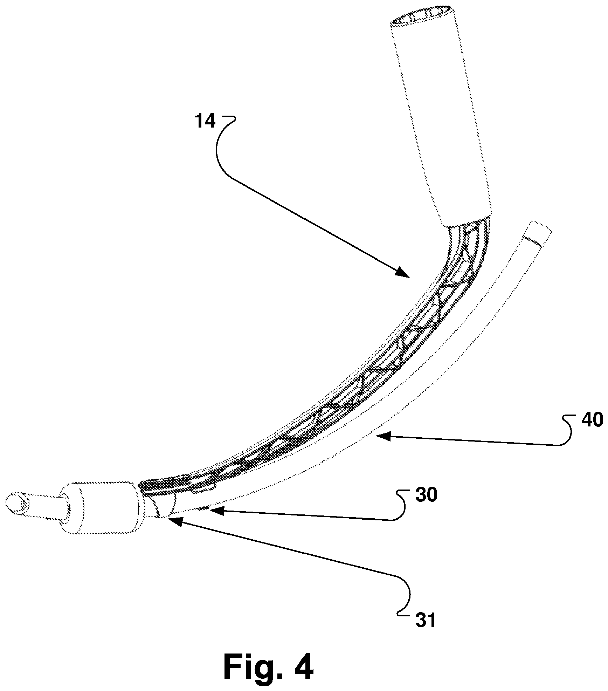

As disclosed in Fig. 3 the first branch is guided/directed along the outside of the endotracheal tube, by the aid of directing means 30, 31, in the distal end of the first branch 14, 24. The directing means 30, 31 is adapted to engagingly grip around the endotracheal tube, such that the first branch 14, 24 is slidably displaceable along the endotracheal tube 40, as disclosed in Fig. 4. The guiding means may be two hooks 30, 31 with the concave surfaces thereof facing each other, as illustrated in Fig. 1. The two hooks 30, 31 may then enclose the endotracheal tube 40 already positioned in the trachea of the patient, whereafter the distal end of the first branch 14 may be slidably displaced along the endotracheal tube 40, into the position of tracheotomy. In this way, the first branch 14 may be controllably inserted into the trachea, without jeopardizing to injure the trachea of the patient. The first branch 14, 24 rests in this case against the front side of the endotracheal tube 40 and will be centered due to the fact that the larynx is triangular with its one apex directed forward.

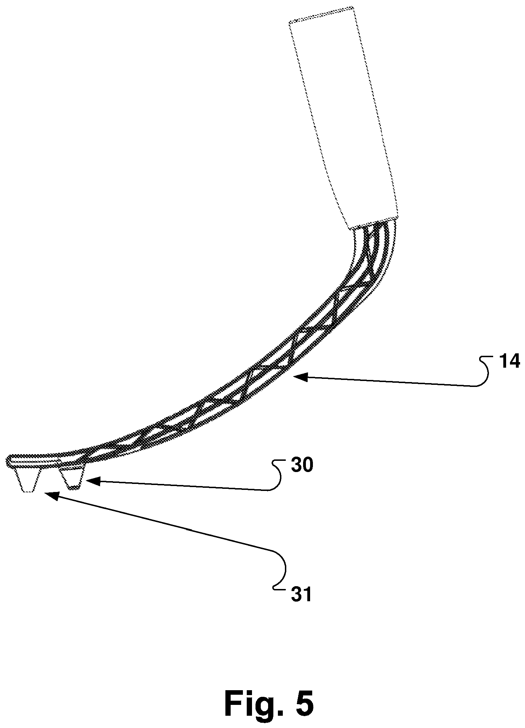

In one embodiment the two hooks 30, 31 may be relatively displaced in the distal end of the first branch 14, as disclosed in Fig. 5. By providing the two hooks 30, 31 relatively displaced, it may be easier to arrange the directing means on the endotracheal tube, while still obtaining satisfactory engagement there between, such that the slidable engagement do not disengage.

The two hooks 30, 31 may, in one embodiment be somewhat flexible, such that the two hooks 30, 31 may snapped around and into engagement with the endotracheal tube.

In one embodiment only one of the hooks 20, 21 is flexible, whereby the same snapping engagement may be achieved.

In yet another embodiment of the device the tracheotomy facilitating device, having a first branch 14 and a second branch 12, further comprises a measurement device in order to provide a determination of the distance from the exterior side of the patients' neck to the inside of the trachea. The measurement device may be a scale fastened between the branches 12, 14. By means of the scale a reading is provided how far the distal ends of device are located from each other. Alternative measurement devices comprise magnetic distance indicator units, optical distance measurement units, etc. Moreover, the distance measurement may directly be performed at the tip of the device (not shown in the figures), e.g. by providing a detectable means in the distal end of inner branch 14, such as a magnetic element or another marker detectable from outside the patient's neck, as for instance by magnetic detection (Hall sensor), ultrasonic detector (reflected sound wave), light detector (of suitable tissue penetrating wavelength), etc. Also this embodiment may be provided with a directing means according to the embodiment disclosed in Figs. 3 to 5.

In another embodiment the two branches 12 or 22, and 14 or 24, respectively, are fixed to each other at connection area. The inner branch 14, 24 is releasably inserteable into an opening at the proximal end of the outer branch 12, 22. Thus the branches 12 or 22, and 14, 24, respectively, may be fastened to each other, either by sliding the inner branch 14, 24 into the outer branch 12, 22, or by sliding the outer branch 12, 22 over the inner branch 14, 24. This embodiment is similar to the embodiment of Figure 2 in this regard. Moreover, a suitable locking unit may be provided for locking the two branches to each other in order to avoid unintended release or misalignments. The branches are provided in a curved design further facilitating application of the device. Inner branch 14, 24 may have an even radius, facilitating introduction down into the trachea, as explained below with reference to the exemplary tracheotomy method. The distal tip of inner branch 14, 24 comprises both a receiving device in form of a cutout, as well as a rounded tip. The tip may be made of a plastic material having low friction, such as PTFE. Also this embodiment may be provided with a directing means according to the embodiment disclosed in Figs. 3 to 5.

Furthermore, inner branch 14, 24 may be of circular diameter. The inner branch 14, 24 may also be hollow, in order to allow gas flow there through.

A gas connector is in fluid communication with the hollow inner of branch 14, 24, leading to a receiving device.

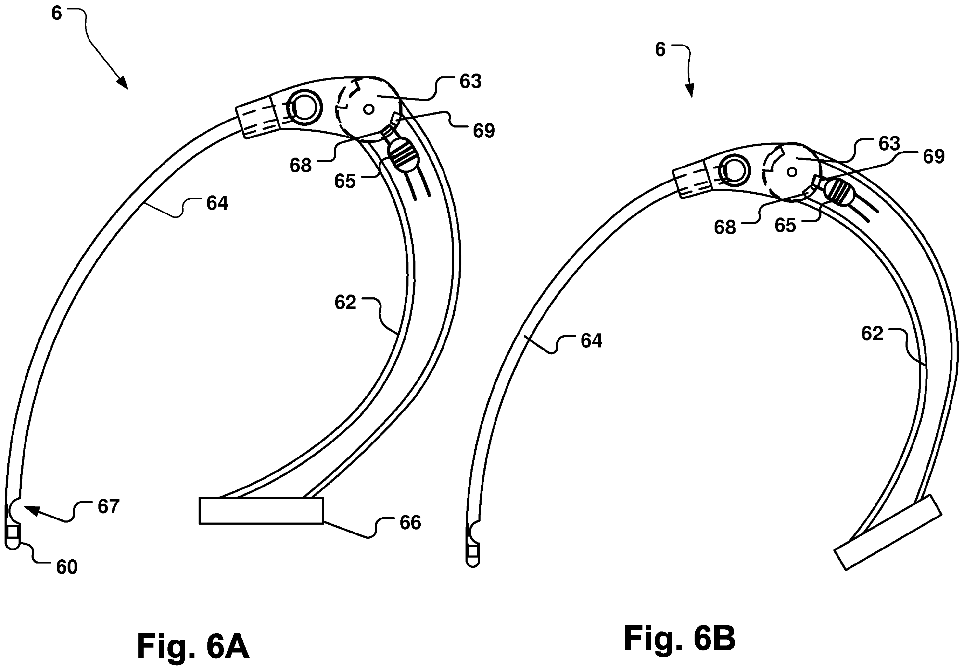

Figure 6A and 6B are schematic illustrations of a further embodiment of the device for facilitating tracheotomy, wherein two states are illustrated, respectively. Figure 6A shows the surgical position, whereas Figure 6B shows the introduction position of device 6. More precisely, the illustrated device 9 has two branches 62, 64 that are pivotable to each other around a joint element 63. The inner branch is inserted into an opening at one proximal end of joint element 63, as shown in Figure 6A. The branches 62, 64 are provided in a curved design further facilitating application of the device 6. However, thanks to the possibility to pivot branches 6, 64 relative each other, the use of device 6 is made even easier. Inner branch 62 may be hollow in order to allow gas flow there through. The distal tip of inner branch 62 comprises both a receiving device 67 in form of a cutout, as well as a rounded tip 60. Tip 60 may be made of a plastic material having low friction, such as PTFE. A gas connector 63 is in fluid communication with the hollow inner of branch 62, leading to receiving device 67. A Y-piece of a respirator may be connected to gas connector 63 by sliding it onto the latter. Also this embodiment may be provided with a directing means according to the embodiment disclosed in Figs. 3 to 5.

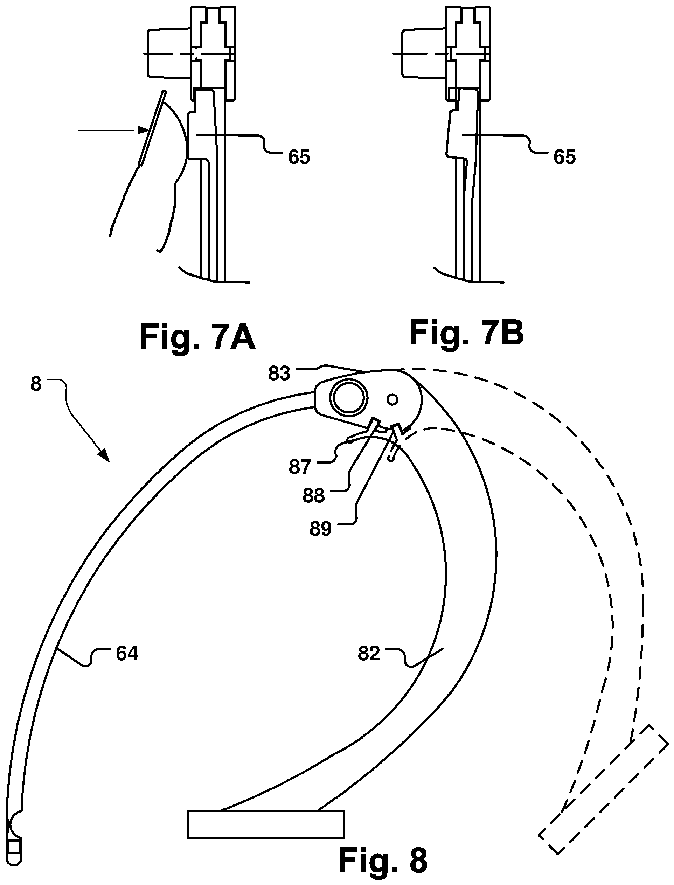

Figure 7A and 7B are schematic illustrations illustrating the release of a locking mechanism comprised in joint element 73 of the device 7 shown in Figure 7A and 7B. The locking mechanism is based on two notches 68, 69, releasably locking the outer arm 62 to the inner arm 64 in predefined positions. A locking member 65 is engaging one of notches 68, 69 in the surgical and the introduction position of device 6, respectively. Fig. 7A illustrates manual manipulation of locking member 65 by a finger of a user, schematically illustrated on the left of this Figure. The finger presses on member 65, pushing it perpendicular to the outer branch 62. The depressed, unlocked position is shown in Fig. 7B. In this position, the two arms 62, 64 are pivotable relative each other between the locking positions. When releasing pressure on locking member 65 it resiliently returns to the initial position, engaging at its end into one of notches 68, 69, thus providing the desired interlocking. Also this embodiment may be provided with a directing means according to the embodiment disclosed in Figs. 3 to 5.

Figure 8 is a schematic illustration illustrating the two states shown in Figure 6A and 6B, respectively, with an alternative locking mechanism. The device 8 is shown in the surgical position by continuous lines, whereas the introduction position of device 8 is shown in dashed lines. More precisely, the illustrated device 8 has two branches 64, 82 that are pivotable to each other around a joint element 63. The inner branch is inserted into an opening at one proximal end of joint element 83. A locking mechanism comprised in joint element 83 of the device 8 comprises two notches 88, 89 arranged on the periphery of joint element 83, as shown in Figure 8. Similar to the embodiment of Figures 6A and 6B, a locking element 87 is releasably locking the outer arm 84 to the inner arm 64 in predefined positions by engaging one of notches 88, 89 in the surgical and the introduction position of device 8, respectively. Locking element 87 is released by drawing it out of a notch. Also this embodiment may be provided with a directing means according to the embodiment disclosed in Figs. 3 to 5.

The tracheostomy facilitating device, such as the above illustrated forceps may be made of a particularly rigid plastic material and be disposable, or of metal for multiple use.

Also, the sharp object, such as a needle, scalpel, etc. may be integrated with the distal end region of one of the branches, e.g. be formed as an integral part with said guiding means.

The sharp object is oriented towards the receiving means and travels on a substantially straight trajectory towards said receiving means.

As mentioned above, the inner branch, which is introduced down along the endotracheal tube may have a ventilation channel. Furthermore, the receiving means located at the distal end of this inner branch may be provided in the form of a bowl formed end, eventually integrated into the branch. The other branch, which is outside of the patient, has at its end a guiding means, such as a holder or guide, e.g. of tubular hollow form, for guiding a puncturing means, such as needle, an obturator or another sharp object suitable for penetrating skin and into the trachea for cutting open the desired opening for the tracheostomy in the patient's neck. The guiding means and the receiving means are oriented towards each other, such that for instance when the puncturing means is guided in the guiding means, it is directed towards the receiving means. Alternatively, the puncturing means may for instance be integrated into the guiding means and thus be directed towards the receiving means together with the guiding means, e.g. on a substantially straight trajectory in a single plane. At the end of puncturing, the puncturing means is thus facing the receiving means. The point or line where the branches virtually meet is the place for the tracheotomy.

The inner branch may be hollow, such that ventilation may be provided through the branch.

Then the inner branch of device is introduced along the outside of the endotracheal tube, as earlier described, by the directing means..

The outer branch is moved towards the neck of the patient and the desired position for the tracheotomy - 2cm above the upper edge of the breastbone / or as far below the vocal cords as desired - may be adjusted by moving the device accordingly along the endotracheal tube, by the aid of guiding means as described above, and consequently outside of the neck, respectively, until the desired position is determined.

The guide, which is provided in the tip of the outer branch, provides guidance of the above-mentioned puncturing device, such as a needle. Needle is moved towards receiving means through guide for puncturing the trachea. The plate of the inner branch may receive the tip of the needle, such that no risk of injuries of the rear wall of the trachea is run. Alternatively, the puncturing device is stopped facing the receiving device, before actually engaging with the receiving device, e.g. by a suitable scale on the puncturing device, or a stop device.

In order to provide a determination of the distance from the exterior side of the patients' neck to the inside of the trachea, a measurement device may be fastened between the branches.

The outer branch is then fold away/removed in order to facilitate the continued dilatation of the tracheal wall for introduction of a trachport. The trachport will serve as a holder for a conventional tracheal cannula.

The above description focuses on embodiments of the present invention applicable to facilitating tracheotomy. However, it will be appreciated that the invention is not limited to this application but may be applied to many other applications. The forceps technique may for example even be used in the following cases:
- Carrying out a fistula through the tracheaoesophagal wall for laryngectomated patients, both primary as well as secondary, for introduction of a voice valve;
- In connection with laterofixation of paralyzed vocal cords. These pair of forceps will have double guides for lead-through of two needles-one above and one below the vocal cords. A fastening suture may then be conveyed through one of the needles and is then forced inside of the other branch and out through the other needle, and when both branches of the pair of forceps have been removed, the suture may be knot together, resulting in the fixation of the vocal cords.

Although the present invention has been described above with reference to specific embodiments, it is not intended to be limited to the specific form set forth herein. Rather, the invention is limited only by the accompanying claims and, other embodiments than the specific above are equally possible within the scope of these appended claims, e.g. different forms of branches than those described above.

In the claims, the term "comprises/comprising" does not exclude the presence of other elements or steps. Furthermore, although individually listed, a plurality of means, elements or method steps may be implemented by e.g. a single unit or processor. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. A device for facilitating tracheotomy (1, 2, 3, 8, 9, 11), comprising:
a first branch (14, 24, 64), adapted to be introduced into the trachea of a patient, and
a second branch (12, 22, 62), adapted to be arranged at the outside of the neck with the distal end thereof located adjacent to a site that is intended for a tracheostoma, wherein
the end of the second branch comprises a guiding means (16, 26, 66) for a sharp object, and
the end of the first branch comprises a receiving means (17, 27, 67), and wherein
the first branch and the second branch are joined to each other in such a manner that the guiding means (16, 26, 66) is oriented towards the receiving means (17, 27, 67),
whereby, when the sharp object penetrates the skin and tracheal wall in order to carry out the tracheostoma, the sharp object is directed towards the receiving means (17, 27, 67)
**characterized in that**
said first branch (14, 24, 64) comprises a directing means (30, 31), adapted to slidingly engage with a tube in trachea, said directing means being located close to the distal end of said first branch (14, 24, 64), for directing said first branch along the outside of said tube.

2. The device according to claim 1, wherein the first branch (14, 24, 64) is adapted to be introduced into the trachea of the patient, such that the distal end thereof is located below the larynx.

3. The device according to claim 1, wherein the second branch (12, 22, 62) is adapted to be arranged at the outside of the neck with the distal end thereof located immediately adjacent to the site that is intended for the tracheostoma.

4. The device according to claim 1, further comprising that the first and second branch are joined to each other by means of a joint (13), such that the branches form a device similar to a pair of forceps, a pair of scissors or a pair of tweezers.

5. The device according to claim 4, wherein said first and second branches are movable relative to each other in order to facilitate the introduction of the first branch down the trachea, wherein the branches are movable relative to each other in a single plane.

6. The device according to claim 1, wherein the second branch (22, 62) is firmly connectable to the first branch (24, 64) after that the first branch is introduced down into the trachea.

7. The device according to any preceding claim, wherein the sharp object (60) is a needle, which is adapted to be introduced through said guiding means, which is a tube, and that the receiving means is a bowl shaped plate.

## Patentansprüche

1. Vorrichtung zum Erleichtern der Tracheotomie (1, 2, 3, 8, 9, 11), umfassend:
einen ersten Schenkel (14, 24, 64), der angepasst ist, in die Trachea eines Patienten eingeführt zu werden, und
einen zweiten Schenkel (12, 22, 62), der angepasst ist, an der Außenseite des Halses angeordnet zu werden, wobei das distale Ende davon benachbart zu einer Stelle positioniert ist, die für ein Tracheostoma bestimmt ist, wobei
das Ende des zweiten Schenkels ein Führungsmittel (16, 26, 66) für einen spitzen Gegenstand umfasst, und
das Ende des ersten Schenkels ein Aufnahmemittel (17, 27, 67) umfasst, und wobei
der erste Schenkel und der zweite Schenkel in einer Weise miteinander verbunden sind, dass das Führungsmittel (16, 26, 66) auf das Aufnahmemittel (17, 27, 67) ausgerichtet ist,
wobei, wenn der spitze Gegenstand durch die Haut und die Trachealwand dringt, um das Tracheostoma hindurchzuführen, der spitze Gegenstand in Richtung des Aufnahmemittels (17, 27, 67) gerichtet ist,
**dadurch gekennzeichnet, dass**
der erste Schenkel (14, 24, 64) eine Leiteinrichtung (30, 31) umfasst, die angepasst ist, gleitend mit einem Tubus in der Luftröhre in Eingriff zu treten, wobei die Leiteinrichtung nahe dem distalen Ende des ersten Schenkels (14, 24, 64) positioniert ist, um den ersten Schenkel entlang der Außenseite des Tubus zu leiten.

2. Vorrichtung nach Anspruch 1, wobei der erste Schenkel (14, 24, 64) angepasst ist, um in die Luftröhre des Patienten derart eingeführt zu werden, dass das distale Ende davon unterhalb des Kehlkopfes positioniert ist.

3. Vorrichtung nach Anspruch 1, wobei der zweite Schenkel (12, 22, 62) angepasst ist, um an der Außenseite des Halses angeordnet zu werden, wobei das distale Ende davon unmittelbar benachbart zu der für das Tracheostoma vorgesehenen Stelle positioniert ist.

4. Vorrichtung nach Anspruch 1, ferner umfassend, dass der erste und der zweite Schenkel mittels eines Gelenks (13) derart miteinander verbunden sind, dass die Schenkel eine Vorrichtung bilden, die einer Zange, einer Schere oder einer Pinzette ähnlich ist.

5. Vorrichtung nach Anspruch 4, wobei der erste und der zweite Schenkel relativ zueinander beweglich sind, um das Einführen des ersten Schenkels in die Luftröhre hinunter zu erleichtern, wobei die Schenkel in einer einzelnen Ebene relativ zueinander beweglich sind.

6. Vorrichtung nach Anspruch 1, wobei der zweite Schenkel (22, 62) fest mit dem ersten Schenkel (24, 64) verbindbar ist, nachdem der erste Schenkel in die Luftröhre hinunter eingeführt wurde.

7. Vorrichtung nach einem der vorhergehenden Ansprüche, wobei der spitze Gegenstand (60) eine Nadel ist, die angepasst ist, durch das Führungsmittel, das ein Tubus ist, eingeführt zu werden, und dass das Aufnahmemittel eine schalenförmige Platte ist.

## Revendications

1. Dispositif pour faciliter une trachéotomie (1, 2, 3, 8, 9, 11), comprenant :
une première branche (14, 24, 64), adaptée pour être introduite dans la trachée d'un patient, et
une seconde branche (12, 22, 62), adaptée pour être agencée à l'extérieur du cou dont l'extrémité distale est située adjacente à un site qui est destiné à un trachéostome, dans lequel
l'extrémité de la seconde branche comprend un moyen de guidage (16, 26, 66) pour un objet pointu, et
l'extrémité de la première branche comprend un moyen de réception (17, 27, 67), et dans lequel
la première branche et la seconde branche sont jointes l'une à l'autre de telle manière que le moyen de guidage (16, 26, 66) soit orienté vers le moyen de réception (17, 27, 67),
moyennant quoi, lorsque l'objet pointu pénètre la peau et la paroi trachéale afin de réaliser le trachéostome, l'objet pointu est dirigé vers le moyen de réception (17, 27, 67)
**caractérisé en ce que**
ladite première branche (14, 24, 64) comprend un moyen de direction (30, 31), adapté pour s'engager en coulissement avec un tube dans la trachée, ledit moyen de direction étant situé près de l'extrémité distale de ladite première branche (14, 24, 64), pour diriger ladite première branche suivant l'extérieur dudit tube.

2. Dispositif selon la revendication 1, dans lequel la première branche (14, 24, 64) est adaptée pour être introduite dans la trachée du patient, de telle sorte que son extrémité distale soit située en dessous du larynx.

3. Dispositif selon la revendication 1, dans lequel la seconde branche (12, 22, 62) est adaptée pour être agencée à l'extérieur du cou dont l'extrémité distale est située immédiatement adjacente au site qui est destiné au trachéostome.

4. Dispositif selon la revendication 1, comprenant en outre le fait que les première et seconde branches soient jointes l'une à l'autre au moyen d'un joint (13), de telle sorte que les branches forment un dispositif similaire à une paire de forceps, une paire de ciseaux ou une paire de pinces.

5. Dispositif selon la revendication 4, dans lequel lesdites première et seconde branches peuvent être déplacées l'une par rapport à l'autre afin de faciliter l'introduction de la première branche en bas de la trachée, dans lequel les branches peuvent être déplacées l'une par rapport à l'autre dans un plan unique.

6. Dispositif selon la revendication 1, dans lequel la seconde branche (22, 62) peut être raccordée solidement à la première branche (24, 64) après que la première branche est introduite vers le bas dans la trachée.

7. Dispositif selon une quelconque revendication précédente, dans lequel l'objet pointu (60) est une aiguille, qui est adaptée pour être introduite à travers ledit moyen de guidage, qui est un tube, et en ce que le moyen de réception est une plaque en forme de bol.
